# EUROPÄISCHE PATENTSCHRIFT

(11) **EP 0 318 682 B1**
(45) Veröffentlichungstag und Bekanntmachung des Hinweises auf die Patenterteilung: **31.03.1993**
(21) Anmeldenummer: 88117164.9
(22) Anmeldetag: 31.10.1985
(51) Int. Cl.: C07D 513/14, C07D 513/04

(54) **Tricyclische Thiazolderivate**
Tricyclic thiazole derivatives
Dérivés tricycliques du thiazole

(30) Priorität: 06.11.1984 CH 5304/84; 05.09.1985 CH 3836/85
(43) Veröffentlichungstag der Anmeldung: 07.06.1989
(62) Teilanmeldung aus: 85113864.4
(73) Patentinhaber: F. HOFFMANN-LA ROCHE AG, 4002 Basel (CH)
(72) Erfinder: Fischer, Ulf, Dr., CH-4402 Frenkendorf (CH); Schneider, Fernand, Dr., CH-4059 Basel (CH); Widmer, Ulrich, Dr., CH-4310 Rheinfelden (CH)
(74) Vertreter: Lederer, Franz, Dr.

(56) Entgegenhaltungen:
- - -

## Beschreibung

Die vorliegende Erfindung betrifft Verbindungen der allgemeinen Formel 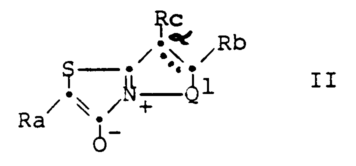
worin Q¹ zusammen mit dem Stickstoffatom eine Gruppe der Formel >N-CH₂CH₂- (a), >N-CH₂CH₂CH₂- (b), >N-CH=CH- (c), >N-CH₂-CH=CH- (d), >N-CH₂-S(O)ₚ-(e), >N-CH₂CH₂-S(O)ₚ- (f) oder >N-CH=CH-S(O)ₚ-(g), p die Zahl 0, 1 oder 2, Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel >C_{α}-S-CH=CH- (h), >C_{α}-CH=CH-S- (i) oder >C_{α}-CH=CH-CH=CH- (j) und die gestrichelte Linie eine zusätzliche Bindung bedeuten. Diese Verbindungen sind Zwischenprodukte zur Herstellung von neuen tricyclischen Pyridonderivaten der allgemeinen Formel 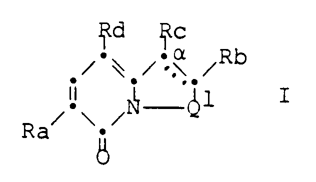
worin Ra, Rb, Rc, Q¹ und die gestrichelte Linie obige Bedeutung besitzen, und Rd Cyano, Nitro oder die Gruppe der Formel -CO-(Q²A²)_{q}-R¹, q die Zahl 0 oder 1, A² niederes Alkylen oder eine direkte Bindung, Q² ein Sauerstoffatom oder die Gruppe -NR²-, R¹ Wasserstoff, Hydroxy, Cyano, Nitro, Halogen, niederes Alkoxy, niederes Alkyl, niederes Alkoxycarbonyl, Aryl, eine Gruppe der Formel -NR³R⁴ oder einen über ein Kohlenstoffatom gebundenen, 5-gliedrigen, gegebenenfalls durch eine oder zwei niedere Alkylgruppen und gegebenenfalls durch eine (C₃₋₆)-Cycloalkyl-, Hydroxy-, niedere Alkoxy-, niedere Alkoxyalkyl-, niedere Alkanoyloxyalkyl-, niedere Alkanoyloxy-, niedere Hydroxyalkyl-, niedere Alkoxycarbonyl-, niedere Alkanoyl-, Carbamoyl-, mono- oder di(niedere Alkyl)carbamoyl-, Oxo- oder Alkylendioxygruppe substituierten, gesättigten, partiell ungesättigten oder aromatischen Heterocyclus, R² Wasserstoff, niederes Alkyl oder Aryl, R³ und R⁴ je Wasserstoff, niederes Alkyl, niederes Alkoxyalkyl, niederes Dialkoxyalkyl, niederes Alkylendioxyalkyl, niederes Cyanoalkyl, niederes Halogenalkyl, niederes Hydroxyalkyl, niederes Dihydroxyalkyl, niederes Alkanoyl, niederes Alkoxycarbonyl oder eine gegebenenfalls durch Hydroxy, niederes Alkoxy, niederes Alkanoyloxy, niederes Hydroxyalkyl, niederes Alkoxyalkyl, niederes Alkanoyloxyalkyl, Oxo, Carbamoyl, mono- oder di(niederes Alkyl)carbamoyl oder durch niederes Alkylendioxy substituierte (C₃₋₇)-Cycloalkylgruppe oder zusammen mit dem Stickstoffatom einen 3- bis 7-gliedrigen, gegebenenfalls durch eine oder zwei niedere Alkylgruppen und gegebenenfalls durch eine oder zwei Hydroxy-, niedere Alkoxy-, niedere Alkanoyloxy-, niedere Hydroxyalkyl-, niedere Alkoxyalkyl-, niedere Alkanoyloxyalkyl-, niedere Alkoxycarbonyl-, niedere Alkanoyl-, Carbamoyl-, mono- oder di(niedere Alkyl)carbamoyl-, Oxo- oder niedere Alkylendioxygruppen substituierten, gesättigten N-Heterocyclus, der als Ringglied noch ein Sauerstoff- oder Schwefelatom oder die Gruppe >N-R⁵ enthalten kann, und R⁵ Wasserstoff, niederes Alkyl, niederes Hydroxyalkyl, niederes Alkoxyalkyl, niederes Alkanoyloxyalkyl, niederes Alkanoyl, niederes Alkoxycarbonyl, Carbamoyl oder mono- oder di(niederes Alkyl)-carbamoyl bedeuten, mit der Massgabe, dass R¹ eine von Hydroxy, Cyano, Nitro, Halogen, niederes Alkoxycarbonyl, niederes Alkoxy und -NR³R⁴ verschiedene Bedeutung hat, wenn q die Zahl 1 und A² eine direkte Bindung bedeuten,
und von pharmazeutisch annehmbaren Säureadditionssalzen von Verbindungen der Formel I mit einem oder mehreren basischen Substituenten.

Diese Verbindungen der Formel I besitzen wertvolle pharmakologische Eigenschaften und können zur Bekämpfung oder Verhütung von Krankheiten verwendet werden. Sie sind insbesondere muskelrelaxierend, sedativhypnotisch, anxiolytisch und/oder antikonvulsiv wirksam und können demnach bei der Bekämpfung oder Verhütung von Muskelspannungen, Spannungszuständen, Schlaflosigkeit, Angstzuständen und/oder Konvulsionen verwendet werden.

Die Verbindungen der Formel I und ihre Herstellung ausgehend von Verbindungen der Formel II werden in der Europäischen Patentanmeldung Nr. 85 113 864.4 (Veröffentlichungsnummer: 183 994) beschrieben.

Der Ausdruck "nieder" bezeichnet Reste und Verbindungen mit höchstens sieben, vorzugsweise höchstens vier Kohlenstoffatomen. Der Ausdruck "Alkyl" für sich allein genommen oder in Kombinationen, wie Alkanoyl, Alkanoyloxy und Alkoxyalkyl, bezeichnet geradkettige oder verzweigte, gesättigte Kohlenwasserstoffreste, wie Methyl, Aethyl, Propyl, Isopropyl und t-Butyl. Der Ausdruck "Cycloalkyl" bezeichnet cyclische, gesättigte Kohlenwasserstoffreste, wie Cyclohexyl. Der Ausdruck "Alkoxy" bezeichnet über ein Sauerstoffatom gebundene Alkylgruppen, wie Methoxy und Aethoxy. Der Ausdruck "Hydroxyalkyl" bezeichnet durch Hydroxy substituierte Alkylgruppen, wie 2-Hydroxyäthyl. die Ausdrücke "Alkanoyl" und "Alkanoyloxy" bezeichnen Fettsäurereste, wie Acetyl und Acetoxy. Der Ausdruck "Alkylen" bezeichnet geradkettige oder verzweigte, gesättigte Kohlenwasserstoffreste mit zwei freien Valenzen, wie Methylen, 1,2-Aethylen und 1,3-Propylen. Der Ausdruck "Halogen" bezeichnet die vier Formen Fluor, Chlor, Brom und Jod.

Der Ausdruck "Aryl" bezeichnet vorzugsweise Phenylgruppen, welche gegebenenfalls durch Halogen, Trifluormethyl, niederes Alkyl, niederes Alkoxy, Nitro, Amino oder mono- oder di(niederes Alkyl)amino substituiert sind.

Die über ein Kohlenstoffatom gebundenen, 5-gliedrigen, gesättigten, partiell ungesättigten oder aromatischen Heterocyclen enthalten als Heteroringglieder vorzugsweise ein Sauerstoff- oder Schwefelatom oder eine Imino- oder niedere Alkyliminogruppe und gegebenenfalls ein oder zwei Stickstoffatome, wobei das Kohlenstoffatom, über das der Heterocyclus gebunden ist, sich vorzugsweise neben einem oder zwischen zwei Heteroatomen befindet. Beispiele solcher Heterocyclen, welche noch wie erwähnt substituiert sein können, sind: 2-Oxazolin-2-yl, 3-Methyl-1,2,4-oxadiazol-5-yl, 2-Thiazolin-2-yl, 2-Tetrahydrofuryl und 2-Thiazolyl.

Der Ausdruck "3- bis 7-gliedriger, gesättigter N-Heterocyclus, der als Ringglied noch ein Sauerstoff- oder Schwefelatom oder die Gruppe >N-R⁵ enthalten kann" als Bedeutungsmöglichkeit für -NR³R⁴ bezeichnet einerseits Heterocyclen mit nur einem Heteroatom, nämlich dem Stickstoffatom, über das sie gebunden sind, und andererseits Heterocyclen mit zwei Heteroatomen, nämlich dem besagten Stickstoffatom und einem Sauerstoff- oder Schwefelatom oder einem zweiten Stickstoffatom. Beispiele derartiger Heterocyclen, welche noch wie erwähnt substituiert sein können, sind: 2-(niederes Alkoxyalkyl)-1-azetidinyl, 3-(niederes Alkoxy)-1-azetidinyl, 3-Hydroxy-1-azetidinyl, 2-(niederes Hydroxyalkyl)-1-azetidinyl, 2-(niederes Alkanoyloxyalkyl)-1-pyrrolidinyl, 3-Oxo-1-pyrrolidinyl, 2-(niederes Alkoxycarbonyl)-1-pyrrolidinyl, 3-(niederes Alkoxy)-1-pyrrolidinyl, 3-Hydroxy-1-pyrrolidinyl, 2-(niederes Alkoxyalkyl)-1-pyrrolidinyl, 2-(niederes Hydroxyalkyl)-1-pyrrolidinyl, 2-(niederes Hydroxyalkyl)-4-hydroxy-1-pyrrolidinyl, 2-(niederes Alkoxyalkyl)-4-(niederes Alkoxy)-1-pyrrolidinyl, 4-Morpholinyl, 2,6-Di(niederes Alkyl)-4-morpholinyl, 4-Thiomorpholinyl, 1-Piperazinyl, 1-(niederes Alkyl)-4-piperazinyl, 1-(niederes Alkoxyalkyl)-4-piperazinyl, 1-(niederes Alkanoyl)-4-piperazinyl, 4-(niederes Hydroxyalkyl)-1-piperidinyl, 4-Oxo-1-piperidinyl, 4-(niederes Alkoxy)-1-piperidinyl, 4-(niederes Alkoxycarbonyl)-1-piperidinyl, 4-Hydroxy-1-piperidinyl, 4-(niederes Alkylcarbamoyl)-1-piperidinyl, 4-(niederes Alkanyloxy)-1-piperidinyl, 2-(niederes Alkoxyalkyl)-1-piperidinyl, 2-(niederes Hydroxyalkyl)-1-piperidinyl, 3-(niederes Alkoxy)-1-piperidinyl, 4,4-(niederes Alkylendioxy)-1-piperidinyl und 3-Hydroxy-1-piperidinyl.

Das Symbol Q¹ bedeutet zusammen mit dem Stickstoffatom vorzugsweise die Gruppe der Formel >N-CH₂CH₂- (a) oder >N-CH=CH- (c). Das Symbol Ra bedeutet vorzugsweise eine gegebenenfalls durch m-Halogen oder m-Trifluormethyl substituierte Phenylgruppe, wobei die Bedeutungsmöglichkeit Phenyl besonders bevorzugt ist. Die Symbole Rb und Rc bedeuten zusammen mit dem mit α bezeichneten Kohlenstoffatom vorzugsweise eine gegebenenfalls durch Halogen substituierte Gruppe der Formel >C_{α}-S-CH=CH- (h) oder >C_{α}-CH=CH-CH=CH- (j), insbesondere die Gruppe der Formel >C_{α}-S-CH=CH- oder >C_{α}-CH=CCl-CH=CH-, wobei die gestrichelte Linie eine zusätzliche Bindung bedeutet.

Die Verbindungen der Formel II können beispielsweise dadurch hergestellt werden, dass man eine Verbindung der allgemeinen Formel 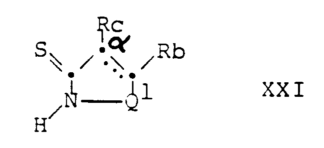
worin Q¹, Rb, Rc und die gestrichelte Linie obige Bedeutung besitzen,
mit einer Verbindung der allgemeinen Formel

Ra-CHX²-COX¹ XXII oder

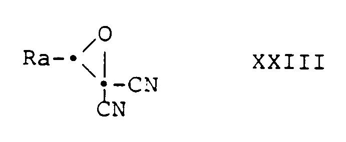

worin X¹ und X² je Halogen bedeuten und Rα obige Bedeutung besitzt,
umsetzt. Die Reaktion einer Verbindung der Formel XXI mit einer Verbindung der Formel XXII, worin X¹ vorzugsweise Chlor und X² vorzugsweise Brom bedeuten, wird vorzugsweise bei Raumtemperatur in einem halogenierten Kohlenwasserstoff, wie Chloroform, durchgeführt, worauf man mit einem basischen Amin, wie Triäthylamin, behandelt. Die Reaktion einer Verbindung der Formel XXI mit einer Verbindung der Formel XXIII wird vorzugsweise in einem inerten Lösungsmittel, wie Aceton, N,N-Dimethylformamid, Dimethylsulfoxid und dergleichen, bei Raumtemperatur durchgeführt.

Die nachfolgenden Beispiele dienen der näheren Erläuterung der vorliegenden Erfindung. Sämtliche Temperaturen sind in Celsiusgraden angegeben.

### Beispiel 1

aa) Methode A: Zu einer Lösung von 23,3 g 4,5-Dihydrothieno[2,3-c]pyridin-7(6H)-thion in 630 ml Chloroform gibt man unter Rühren tropfenweise 29,4 g α-Bromphenylessigsäurechlorid, wobei man dafür sorgt, dass die Temperatur nicht über 25° steigt. Nach 30 Minuten versetzt man mit 25,45 g Triäthylamin und rührt noch während 3 Stunden. Man verdünnt die Mischung mit Wasser, trennt die organische Phase ab, wäscht mit gesättigter wässriger Natriumchloridlösung, trocknet über Magnesiumsulfat, dampft ein und chromatographiert den Rückstand an Kieselgel (Laufmittel Toluol/Aethanol 95:5). Man erhält 5,6-Dihydro-3-hydroxy -2-phenylthiazolo[3,2-a]thieno[2,3-c]pyridinium-hydroxid (inneres Salz) vom Smp. 225° (Zers.) (aus Dioxan/Acetonitril).

Methode B: Zu einer Lösung von 26,35 g 4,5-Dihydrothieno[2,3-c]pyridin-7(6H)-thion in 140 ml Dimethylformamid gibt man unter Rühren 28,6 g 1-Phenyl-2,2-dicyan-oxiran, wobei sich die Lösung sofort tiefrot färbt. Nach 16 Stunden wird der kristalline Niederschlag abgesaugt und mit Essigester gewaschen. Man erhält 5,6-dihydro-3-hydroxy-2-phenylthiazolo[3,2-a]thieno[2,3-c]pyridinium-hydroxid (inneres Salz) vom Smp. 208-209°.

In analoger Weise erhält man:
ab) Aus 4,5-Dihydrothieno[2,3-c]pyridin-7(6H)-thion und 1-(o-Chlorphenyl)-2,2-dicyan-oxiran das 5,6-Dihydro-3-hydroxy -2-(o-chlorphenyl)-thiazolo[3,2-a]thieno[2,3-c]pyridinium -hydroxid (inneres Salz) vom Smp. 174-175° (aus Dioxan/Acetonitril);
ac) aus 4,5-Dihydrothieno[2,3-c]pyridin-7(6H)-thion und 1-(m-Chlorphenyl)-2,2-dicyan-oxiran das 5,6-Dihydro-3-hydroxy -2-(m-chlorphenyl)-thiazolo[3,2-a]thieno[2,3-c]pyridinium -hydroxid (inneres Salz) vom Smp. 179-181°;
ad) aus 4,5-Dihydrothieno[2,3-c]pyridin-7(6H)-thion und 1-(p-Chlorphenyl)-2,2-dicyan-oxiran das 5,6-Dihydro-3hydroxy -2-(p-chlorphenyl)-thiazolo[3,2-a]thieno[2,3-c]pyridinium -hydroxid (inneres Salz) vom Smp. 215° (Zers.);
ae) aus 4,5-Dihydrothieno[2,3-c]pyridin-7(6H)-thion und 1-(m-Chlorphenyl)-2,2-dicyan-oxiran das 5,6-Dihydro-3hydroxy -2-(m-chlorphenyl)-thiazolo[3,2-a]thieno[2,3-c]pyridinium -hydroxid (inneres Salz);
af) aus 6,7-Dihydrothieno[3,2-c]pyridin-7(6H)-thion mit 1-Phenyl-2,2-dicyan-oxiran das 5,6-Dihydro-3-hydroxy -2-phenylthiazolo[3,2-a]thieno[3,2-c]pyridinium -hydroxid (inneres Salz) vom Smp. 198-202° (Zers.)

### Beispiel 2

aa) Methode A: 23,65 g 3,4-Dihydroisochinolin-1(2H)-thion werden in 750 ml Chloroform gelöst, worauf man bei Raumtemperatur unter Kühlen zunächst mit 54 g α-Bromphenylacetylchlorid und 90 Minuten später mit 53 ml Triäthylamin versetzt und über Nacht bei Raumtemperatur rührt. Das Reaktionsgemisch wird mit Wasser gewaschen, über Magnesiumsulfat getrocknet und im Vakuum eingedampft. Man erhält 5,6-Dihydro-3-hydroxy -2-phenylthiazolo[2,3-a]isochinolinium-hydroxid (inneres Salz) vom Smp. 210° (Zers.) (aus Acetonitril/Dioxan).
ab) Methode B: 2,96 g 7-Chlor-3,4-dihydroisochinolin-1(2H)thion und 3,7 g 1-(p-Chlorphenyl)-2,2-dicyanoxiran werden in 60 ml Aceton über Nacht gerührt. Der ausgefallene rote kristalline Niederschlag wird abgesaugt und aus Dioxan umkristallisiert. Man erhält 9-Chlor-2-(p-chlorphenyl)-5,6-dihydro -3-hydroxythiazolo[2,3-a]isochinolinium-hydroxid (inneres Salz) vom Smp. 276° (Zers.).
   In analoger Weise erhält man:
ac) Aus 7-Chlor-3,4-dihydroisochinolin-1(2H)-thion und α-Bromphenylacetylchlorid (Methode A) oder 1-Phenyl-2,2-dicyanoxiran (Methode B) das 9-Chlor-2-phenyl-5,6-dihydro -3-hydroxythiazolo[2,3-a]isochinolinium-hydroxid (inneres Salz) vom Smp. 296° (Zers.);
ad) aus 7-Chlor-3,4-dihydroisochinolin-1(2H)-thion und 1-(o-Chlorphenyl)-2,2-dicyanoxiran das 9-Chlor-2-(o-chlorphenyl)-5,6-dihydro -3-hydroxythiazolo[2,3-a]isochinolinium-hydroxid (inneres Salz) vom Smp. 260-262° (Methode B, Dimethylformamid als Lösungsmittel).

### Beispiel 3

aaa) 38,9 g 3,3′-Dithiobis-thiophen-2-carbonsäure-methylester werden in 1500 ml 1N-Natronlauge und 700 ml Aethanol suspendiert, worauf man bis zur Beendigung der Reaktion unter Rückfluss erhitzt. Nach Abkühlen im Eisbad wird mit 25-proz. Salzsäure angesäuert, wobei das Produkt auskristallisiert. Durch Umkristallisieren aus Wasser erhält man 3,3′-Dithio-bis-thiophen-2-carbonsäure als farblose Kristalle vom Smp. 256-257°.
aab) 41,3 g 3,3′-Dithiobis-thiophen-2-carbonsäure werden in 340 ml Thionylchlorid suspendiert, worauf man während 2,5 Stunden unter Rückfluss erhitzt. Das überschüssige Thionylchlorid wird im Vakuum entfernt, und der Rückstand wird in 700 ml Dioxan suspendiert. Es wird nun Ammoniak in die Lösung eingeleitet. Nach Kühlen im Eisbad wird filtriert, und die Kristalle werden in 1500 ml Wasser während 30 Minuten gerührt. Die Kristalle werden abfiltriert. Durch Umkristallisieren aus n-Butanol/Dioxan erhält man 3,3′-Dithiobis-thiophen-2-carboxamid als farblose Kristalle vom Smp. 222-223°.
aac) 77,5 g 3,3′-Dithio-bis-thiophen-2-carboxamid werden in 1200 ml Dioxan suspendiert, worauf man unter Inertgas auf 45° erwärmt. Zur Suspension gibt man bei 45-48° portionenweise 46,4 g Natriumborhydrid. Nach Beendigung der Wasserstoffentwicklung wird bis zum Verschwinden des Disulfides unter Rückfluss erhitzt. Unter Kühlen werden nun 450 ml Wasser zugetropft und anschliessend wird mit 2N-Salzsäure angesäuert. Nach Verdünnen der Lösung mit 1800 ml Wasser und Sättigung mit Kochsalz wird erschöpfend mit Aether extrahiert. Die vereinigten Extrakte werden über Natriumsulfat getrocknet, filtriert und eingedampft. Nach Trocknen erhält man das rohe 3-Mercaptothiophen-2-carboxamid vom Smp. 116-120°.
aad) 11,1 g 3-Mercapto-thiophen-2-carboxamid, 2,2 g 95-proz. Paraformaldehyd und 13,3 g p-Toluolsul- fonsäure-monohydrat werden zusammen mit 300 ml Mesitylen unter Rückfluss erhitzt. Nach beendeter Reaktion lässt man abkühlen und entfernt das Lösungsmittel im Vakuum. Nach Chromatographieren des Rückstandes an Kieselgel und Umkristallisieren aus Essigsäureäthylester erhält man 2,3-Dihydro-4H-thieno[2,3-e][1,3]thiazin-4-on als gelbliche Kristalle vom Smp. 153-155°.
aae) 19,6 g 2,3-Dihydro-4H-thieno[2,3-e][1,3]-thiazin-4-on werden zusammen mit 23,1 g Lawesson-Reagens in 200 ml Acetonitril unter Rückfluss solange erhitzt, bis alles Ausgangsmaterial umgesetzt ist. Das Reaktionsgemisch wird abgekühlt, und das auskristallisierte Produkt wird abfiltriert. Durch Umkristallisieren aus Essigsäureäthylester erhält man reines 2,3-Dihydro-4H-thieno[2,3-e][1,3]thiazin-4-thion vom Smp. 136-138°. Durch Eindampfen der Mutterlauge und Umkristallisieren kann weiteres Material gewonnen werden.
aba) In analoger Weise erhält man aus 4,5,6,7-Tetrahydro-8H-thieno[2,3-c]azepin-8-on das 4,5,6,7-Tetrahydro-8H-thieno[2,3-c]azepin-8-thion vom Smp. 103-104° (aus Essigsäureäthylester).
aca) Methode A: 17,4 g 3-Mercapto-thiophen-2-carbonsäure-methylester werden unter einer Inertgasatmosphäre in 350 ml Toluol gelöst, worauf man mit 20,5 g 2-Aminoäthylbromid-hydrobromid und dann mit 100 ml 3N-Natriummethylatlösung in Methanol versetzt. Es wird während 30 Minuten bei Raumtemperatur gerührt und nachher unter Rückfluss erhitzt, bis die Reaktion beendet ist. Man dampft im Vakuum ein, nimmt den Rückstand in 300 ml Wasser auf, säuert mit 2N-Salzsäure an und filtriert die Kristalle ab. Durch Umkristallisieren aus Chloroform erhält man 3,4-Dihydro-thieno[2,3-f][1,4]thiazepin-5(2H)-on als weisse Kristalle vom Smp. 186-188°.

Methode B: 65,4 g 3-Mercapto-thiophen-2-carbonsäure-methylester werden in 800 ml Aethanol gelöst, worauf man mit 19,4 ml Aethylenimin versetzt. Nach beendeter Reaktion wird das Reaktionsgemisch im Eisbad abgekühlt, und durch Einleiten von trockenem Chlorwasserstoff wird das Hydrochlorid ausgefällt. Die Kristalle werden abfiltriert, und das Filtrat wird eingedampft, wobei noch mehr Rohprodukt gewonnen wird. Durch Umkristallisieren aus Methanol/Essigsäureäthylester erhält man 3-[(2-Aminoäthyl)thio]-2-thiophen-carbonsäure-methylester -hydrochlorid als weisse Kristalle vom Smp. 174-176°.

1,0 g 3-[(2-Aminoäthyl)thio]-2-thiophen-carbonsäure-methylester - hydrochlorid werden unter Argon in 20 ml Toluol suspendiert, worauf man 9,4 Ml 1N-Natriummethylatlösung in Methanol zugibt. Nach beendeter Reaktion wird im Vakuum eingedampft, und der Rückstand wird mit 20 ml Wasser behandelt. Die Kristalle werden abfiltriert, mit Wasser gewaschen und getrocknet. Durch Umkristallisieren erhält man 3,4-Dihydro-thieno[2,3-f][1,4]thiazepin-5(2H)-on als weisse Kristalle vom Smp. 186-188°. Die Reaktion kann auch mit 1,1 Moläquivalent Kalium-tert-butylatlösung in Toluol ausgeführt werden.
acb) 1,0 g 3,4-Dihydro-thieno[2,3-f][1,4]thiazepin-5(2H)-on werden zusammen mit 15 ml Pyridin und 1,35 g Phosphorpentasulfid während 5 Stunden unter Rückfluss erhitzt. Nach Abkühlen giesst man auf 90 ml Wasser und filtriert die Kristalle ab. Nach Chromatographieren an Kieselgel wird aus Essigsäureäthylester ukristallisiert. Man erhält 3,4-Dihydro-thieno[2,3-f][1,4]thiazepin-5(2H)-thion als gelbe Kristalle vom Smp. 138-139°.
ada) 288 g Thieno[2,3-c]pyridin werden in 5,3 l Methylenchlorid gelöst und bei -5° bis 0° wird portionenweise eine äquivalente Menge m-Chlorperbenzoesäure zugesetzt. Es wird bis zur Beendigung der Reaktion weitergerührt. Zugabe von 800 ml gesättigter ätherischer Chlorwasserstofflösung fällt das Produkt als weisse Kristalle aus, die mit Aether gewaschen werden. Das so erhaltene Thieno[2,3-c]pyridin 6-oxid-hydrochlorid ist genügend rein, um für die nächste Stufe ein- gesetzt zu werden, Smp. 202°.
adb) 671,5 g Thieno[2,3-c]pyridin 6-oxid-hydrochlorid werden unter Argon in 4000 ml Dioxan suspendiert und 655 ml Phosphoroxychlorid werden zugesetzt. Es wird auf dem Oelbad bis zum Einsetzen der exothermen Reaktion erwärmt. Nach dem Abklingen der exothermen Reaktion wird noch 10 Minuten zum Rückfluss erhitzt. Man engt im Vakuum ein und nimmt den Rückstand in 2000 ml Toluol auf. Dann wird unter Kühlung langsam mit 3000 ml Wasser versetzt. Durch portionenweise Zugabe von Soda wird neutralisiert. Die organische Phase wird abgetrennt und die wässrige Phase nochmals mit 1000 ml Toluol extrahiert. Nach Waschen mit Wasser wird über Natriumsulfat getrocknet, filtriert und eingedampft, wobei rohes 7-Chlor-thieno[2,3-c]pyridin als braunes Oel erhalten wird.
adc) 4,85 g 7-Chlor-thieno[2,3-c]pyridin werden in 15 ml Dimethylformamid gelöst und unter Argon werden 3,18 g Natriumhydrogensulfid-monohydrat zugesetzt. Es wird eine Stunde auf 110-115° erhitzt, nochmals 1,06 g Natriumhydrogensulfid-monohydrat zusetzt und während einer weiteren Stunde bei der oben angegebenen Temperatur hält. Es wird auf 150 ml Eiswasser gegossen und mit 1N wässeriger Salzsäurelösung angesäuert. Nach kurzem Rühren bei 2° werden die gelblichen Kristalle abfiltriert. Nach Umkristallisation aus einem Toluol/Essigsäureäthylestergemisch erhält man reines Thieno[2,3-c]pyridin-7(6H)-thion, Smp. 187-189°.
ba) 16,7 g Thieno[2,3-c]pyridin-7(6H)-thion werden in 1000 ml Methylenchlorid unter einer Inertgasatmosphäre suspendiert, worauf man 15,3 ml ca. 90-proz. α-Bromphenylessigsäurechlorid zutropft. Nach beendeter Zugabe wird noch etwa eine halbe Stunde bei Raumtemperatur gerührt und dann 27,8 ml Triäthylamin zugetropft. Anschliessend wird während 30 Minuten bei Raumtemperatur gerührt. Die Lösung wird zweimal mit je 750 ml Wasser gewaschen, über Natriumsulfat getrocknet, filtriert und im Vakuum eingedampft. Die erhaltenen roten Kristalle werden durch Chromatographieren und Umkristallisieren aus Chloroform/Aether/Hexan gereinigt. Man erhält 3-Hydroxy-2-phenylthiazolo[3,2-a]thieno[2,3-c]pyridinium-hydroxid (inneres Salz) als rote Kristalle vom Smp. 195-200° (Zers.).
   In analoger Weise erhält man:
bb) Aus 2,3-Dihydro-4H-thieno[2,3-e][1.3]thiazin-4-thion und α-Bromphenacetylchlorid das 7-Hydroxy-8-phenyl-5H-thiazolo[3,2-c]thieno[2,3-e][1,3] thiazinium-hydroxid (inneres Salz) vom Smp. 194-197° (aus Methanol);
bc) aus 4,5,6,7-Tetrahydro-8H-thieno[2,3-c]azepin-8-thion und α-Bromphenacetylchlorid das 5,6-Dihydro-8-hydroxy-9-phenyl -4H-thiazolo[3,2-a]thieno[2,3-c]azepinium-hydroxid (inneres Salz) vom Smp. 205-208° (aus Chloroform/Diäthyläther);
bd) aus 3,4-Dihydrothieno[2,3-f][1,4]thiazepin-5(2H)-thion und α-Bromphenacetylchlorid das 5,6-Dihydro-8-hydroxy-9-phenyl -thiazolo[3,2-d]thieno[2,3-f][1,4]thiazepinium-hydroxid (inneres Salz) vom Smp. 196-198° (aus Chloroform/Diäthyläther).

### Beispiel 4

Die in Beispiel 3bd beschriebene Verbindung kann auch wie folgt hergestellt werden:
0,6 g 3,4-Dihydro-thieno[2,3-f][1,4]thiazepin-5(2H)-thion und 1,0 g α-(Tosylhydrazono)phenylacetyl-chlorid werden in 50 ml Methylenchlorid längere Zeit bei Raumtemperatur und in Gegenwart von 0,84 ml Triäthylamin gerührt. Das Lösungsmittel wird im Vakuum entfernt, und der Rück- stand wird an Kieselgel chromatographiert. Man erhält 5,6-Dihydro-8-hydroxy-9-phenyl -thiazolo[3,2-d]thieno[2,3-f][1,4]thiazepinium-hydroxyd (inneres Salz) vom Smp. 190-195°.

## Patentansprüche (Patentansprüche für folgende(n) Vertragsstaat(en): E, CH, DE, FR, IT, LI, LU, NL, SE)

1. Verbindungen der allgemeinen Formel 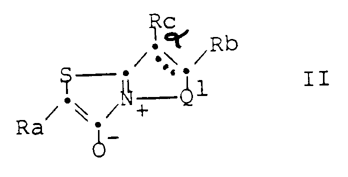 worin Q¹ zusammen mit dem Stickstoffatom eine Gruppe der Formel >N-CH₂CH₂- (a), >N-CH₂CH₂CH₂- (b), >N-CH=CH- (c), >N-CH₂-CH=CH- (d), >N-CH₂-S(O)ₚ-(e), >N-CH₂CH₂-S(O)ₚ- (f) oder >N-CH=CH-S(O)ₚ-(g), p die Zahl 0, 1 oder 2, Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel >C_{α}-S-CH=CH- (h), >C_{α}-CH=CH-S- (i) oder >C_{α}-CH=CH-CH=CH- (j) und die gestrichelte Linie eine zusätzliche Bindung bedeuten, und die mit nieder bezeichneten Reste höchstens 7 Kohlenstoffatome besitzen.

2. Verbindungen nach Anspruch 1, worin Q¹ zusammen mit dem Stickstoffatom die Gruppe der Formel >N-CH₂CH₂- (a) oder >N-CH=CH- (c) bedeutet.

3. Verbindungen nach Anspruch 1 oder 2, worin Ra eine gegebenenfalls durch m-Halogen oder m-Trifluormethyl substituierte Phenylgruppe bedeutet.

4. Verbindungen nach Anspruch 3, worin Ra Phenyl bedeutet.

5. Verbindungen nach einem der Ansprüche 1-4, worin Rb und Rc zusammen mit dem mit α bezeichneten Kohlenstoffatom eine gegebenenfalls durch Halogen substituierte Gruppe der Formel >C_{α}-S-CH=CH- oder >C_{α}-CH=CH-CH=CH- und die gestrichelte Linie eine zusätzliche Bindung bedeuten.

6. Verbindungen nach Anspruch 5, worin Rb und Rc zusammen mit dem mit α bezeichneten Kohlenstoffatom die Gruppe der Formel >C_{α}-S-CH=CH- oder >C_{α}-CH=CCl-CH=CH- bedeuten.

7. 5,6-Dihydro-3-hydroxy-2-phenylthiazolo[3,2-a]thieno[2,3-c]pyridinium-hydroxid (inneres Salz).

8. 9-Chlor-2-phenyl-5,6-dihydro-3-hydroxythiazolo[2,3-a]isochinolinium-hydroxid (inneres Salz).

9. Verfahren zur Herstellung von Verbindungen nach einem der Ansprüche 1-8, dadurch gekennzeichnet, dass man eine Verbindung der allgemeinen Formel 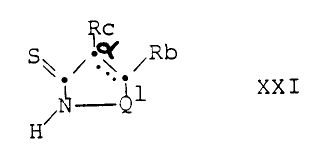 worin Q¹, Rb, Rc und die gestrichelte Linie die in Anspruch 1 angegebene Bedeutung besitzen,
mit einer Verbindung der allgemeinen Formel
Ra-CHX²-COX¹ XXII oder 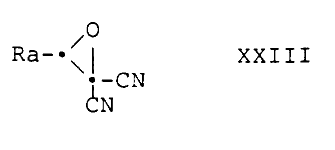
worin X¹ und X² je Halogen bedeuten, und Rα die in Anspruch 1 angegebene Bedeutung besitzt,
umsetzt.

## Patentansprüche (Patentansprüche für folgende(n) Vertragsstaat(en): AT)

1. Verfahren zur Herstellung von Verbindungen der allgemeinen Formel 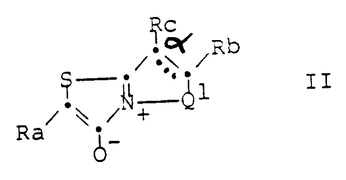 worin Q¹ zusammen mit dem Stickstoffatom eine Gruppe der Formel >N-CH₂CH₂- (a), >N-CH₂CH₂CH₂- (b), >N-CH=CH- (c), >N-CH₂-CH=CH- (d), >N-CH₂-S(O)ₚ-(e), >N-CH₂CH₂-S(O)ₚ- (f) oder >N-CH=CH-S(O)ₚ-(g), p die Zahl 0, 1 oder 2, Ra eine gegebenenfalls durch Halogen, Trifluormethyl, Nitro, niederes Alkyl oder niederes Alkoxy substituierte Phenyl-, Pyridyl- oder Thienylgruppe, Rb und Rc zusammen mit dem mit α bezeichneten Kohlenstoffatom eine gegebenenfalls durch Halogen, Trifluormethyl, niederes Alkyl, niederes Alkoxy, Nitro, Amino oder mono- oder di(niederes Alkyl)amino substituierte Gruppe der Formel >C_{α}-S-CH=CH- (h), >C_{α}-CH=CH-S- (i) oder >C_{α}-CH=CH-CH=CH- (j) und die gestrichelte Linie eine zusätzliche Bindung bedeuten, und die mit nieder bezeichneten Reste höchstens 7 Kohlenstoffatome besitzen,
dadurch gekennzeichnet, dass man eine Verbindung der allgemeinen Formel 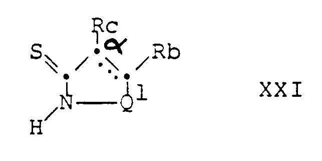 worin Q¹, Rb, Rc und die gestrichelte Linie obige Bedeutung besitzen,
mit einer Verbindung der allgemeinen Formel
Ra-CHX²-COX¹ XXII oder 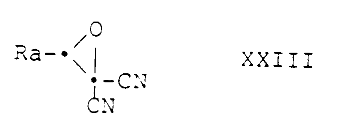
worin X¹ und X² je Halogen bedeuten und Rα obige Bedeutung besitzt,
umsetzt.

2. Verfahren nach Anspruch 1, worin Q¹ zusammen mit dem Stickstoffatom die Gruppe der Formel >N-CH₂CH₂- (a) oder >N-CH=CH- (c) bedeutet.

3. Verfahren nach Anspruch 1 oder 2, worin Ra eine gegebenenfalls durch m-Halogen oder m-Trifluormethyl substituierte Phenylgruppe bedeutet.

4. Verfahren nach Anspruch 3, worin Ra Phenyl bedeutet.

5. Verfahren nach einem der Ansprüche 1-4, worin Rb und Rc zusammen mit dem mit α bezeichneten Kohlenstoffatom eine gegebenenfalls durch Halogen substituierte Gruppe der Formel >C_{α}-S-CH=CH- oder >C_{α}-CH=CH-CH=CH- und die gestrichelte Linie eine zusätzliche Bindung bedeuten.

6. Verfahren nach Anspruch 5, worin Rb und Rc zusammen mit dem mit α bezeichneten Kohlenstoffatom die Gruppe der Formel >C_{α}-S-CH=CH- oder >C_{α}-CH=CCl-CH=CH- bedeuten.

7. Verfahren nach Anspruch 1, dadurch gekennzeichnet, dass man 5,6-Dihydro-3-hydroxy-2-phenylthiazolo[3,2-a]thieno[2,3-c]pyridinium-hydroxid (inneres Salz)herstellt.

8. Verfahren nach Anspruch 1, dadurch gekennzeichnet, dass man 9-Chlor-2-phenyl-5,6-dihydro-3-hydroxythiazolo[2,3-a]isochinolinium-hydroxid (inneres Salz)herstellt.

## Claims (Claims for the following Contracting State(s): BE, CH, DE, FR, IT, LI, LU, NL, SE)

1. Compounds of the general formula 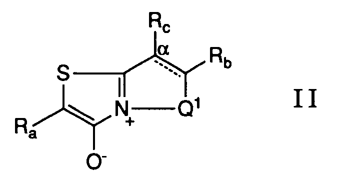 wherein Q¹ and the nitrogen atom together signify a group of the formula >N-CH₂CH₂- (a), >N-CH₂CH₂CH₂- (b), >N-CH=CH- (c), >N-CH₂-CH=CH- (d), >N-CH₂-S(O)ₚ (e), >N-CH₂CH₂-S(O)ₚ- (f), or >N-CH=CH-S(O)ₚ- (g), p signifies the number 0, 1 or 2, Rₐ signifies a phenyl, pyridyl or thienyl group which is optionally substituted by halogen, trifluoromethyl, nitro, lower alkyl or lower alkoxy, R_{b} and R_{c} together with the carbon atom denoted by α signify a group of the formula >C_{α}-S-CH=CH- (h), >C_{α}-CH=CH-S- (i) or >C_{α}-CH=CH-CH=CH- (j) which is optionally substituted by halogen, trifluoromethyl, lower alkyl, lower alkoxy, nitro, amino or mono- or di(lower alkyl)amino, and the dotted line signifies an additional bond, and the residues denoted as lower have a maximum of seven carbon atoms.

2. Compounds according to claim 1, wherein Q¹ and the nitrogen atom together signify the group of the formula >N-CH₂-CH₂- (a) or >N-CH=CH- (c).

3. Compounds according to claim 1 or 2, wherein Rₐ signifies a phenyl group which is optionally substituted by m-halogen or m-trifluoromethyl.

4. Compounds according to claim 3, wherein Rₐ signifies phenyl.

5. Compounds according to any one of claims 1 to 4, wherein R_{b} and R_{c} together with the carbon atom denoted by α signify a group of the formula >C_{α}-S-CH=CH- or >C_{α}-CH=CH-CH=CH- which is optionally substituted by halogen and the dotted line signifies an additional bond.

6. Compounds according to claim 5, wherein R_{b} and R_{c} together with the carbon atom denoted by α signify the group of the formula >C_{α}-S-CH=CH- or >C_{α}-CH=CCl-CH=CH-.

7. 5,6-Dihydro-3-hydroxy-2-phenylthiazolo[3,2-a]thieno[2,3-c]pyridinium hydroxide (internal salt).

8. 9-Chlor-2-phenyl-5,6-dihydro-3-hydroxythiazolo[2,3-a]isoquinolinium hydroxide (internal salt).

9. A process for the manufacture of compounds according to any one of claims 1 to 8, characterized by reacting a compound of the general formula 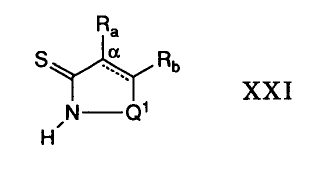 wherein Q¹, R_{b}, R_{c} and the dotted line have the significance given in claim 1 with a compound of the general formula 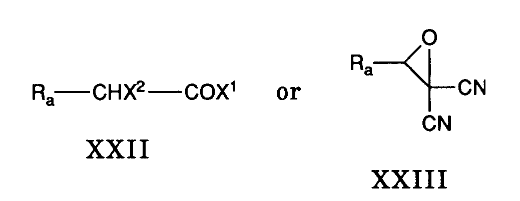 wherein X¹ and X² respectively signifies halogen and Rₐ has the significance given in claim 1.

## Claims (Claims for the following Contracting State(s): AT)

1. A process for the manufacture of compounds of the general formula 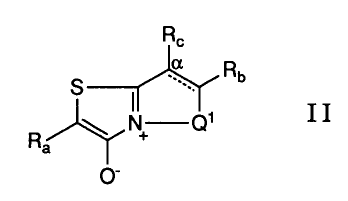 wherein Q¹ and the nitrogen atom together signify a group of the formula >N-CH₂CH₂- (a), >N-CH₂CH₂CH₂- (b), >N-CH=CH- (c), >N-CH₂-CH=CH- (d), >N-CH₂-S(O)ₚ (e), >N-CH₂CH₂-S(O)ₚ- (f), or >N-CH=CH-S(O)ₚ- (g), p signifies the number 0, 1 or 2, Rₐ signifies a phenyl, pyridyl or thienyl group which is optionally substituted by halogen, trifluoromethyl, nitro, lower alkyl or lower alkoxy, R_{b} and R_{c} together with the carbon atom denoted by α signify a group of the formula >C_{α}-S-CH=CH- (h), >C_{α}-CH=CH-S- (i) or >C_{α}-CH=CH--CH=CH- (j) which is optionally substituted by halogen, trifluoromethyl, lower alkyl, lower alkoxy, nitro, amino or mono- or di(lower alkyl)amino, and the dotted line signifies an additional bond, and the residues denoted as lower have a maximum of seven carbon atoms, characterized by reacting a compound of the general formula 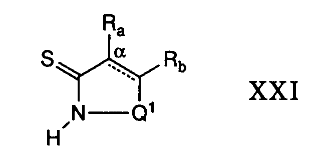 wherein Q¹, R_{b}, R_{c} and the dotted line have the above significance, with a compound of the general formula 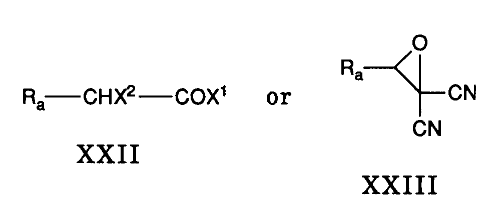 wherein X¹ and X² respectively signifies halogen and Rₐ has the above significance.

2. A process according to claim 1, wherein Q¹ and the nitrogen atom together signify the group of the formula >N-CH₂-CH₂- (a) or >N-CH=CH- (c).

3. A process according to claim 1 or 2, wherein Rₐ signifies a phenyl group which is optionally substituted by m-halogen or m-trifluoromethyl.

4. A process according to claim 3, wherein Rₐ signifies phenyl.

5. A process according to any one of claims 1 to 4, wherein R_{b} and R_{c} together with the carbon atom denoted by α signify a group of the formula >C_{α}-S-CH=CH- or >C_{α}-CH=CH-CH=CH- which is optionally substituted by halogen and the dotted line signifies an additional bond.

6. A process according to claim 5, wherein R_{b} and R_{c} together with the carbon atom denoted by α signify the group of the formula >C_{α}-S-CH=CH- or >C_{α}-CH=CCl-CH=CH-.

7. A process according to claim 1, characterized in that 5,6-Dihydro-3-hydroxy-2-phenylthiazolo[3,2-a]thieno[2,3-c]pyridinium hydroxyide (internal salt) is manufactured.

8. A process according to claim 1, characterized in that 9-Chloro-2-phenyl-5,6-dihydro-3-hydroxythiazolo[2,3-a]isoquinolinium hydroxide (internal salt) is manufactured.

## Revendications (Revendications pour l'(les) Etat(s) contractant(s) suivant(s): BE, CH, DE, FR, IT, LI, LU, NL, SE)

1. Composés de formule générale 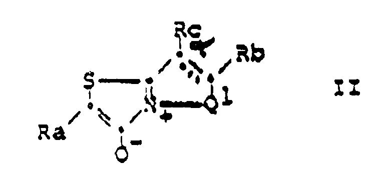 dans laquelle Q¹ représente avec l'atome d'azote un groupe de formule >N-CH₂CH₂- (a), >N-CH₂CH₂CH₂- (b), >N-CH=CH- (c), >N-CH₂-CH=CH- (d), >N-CH₂-S(O)ₚ- (e), >N-CH₂CH₂-S(O)ₚ- (f) ou >N-CH=CH-S(O)ₚ- (g), p représente le nombre 0, 1 ou 2, Ra un groupe phényle, pyridyle ou thiényle éventuellement substitué par un halogène, un trifluorométhyle, un nitro, un alcoyle inférieur ou un alcoxy inférieur, Rb et Rc représentent ensemble aveu l'atome de carbone désigné par α un groupe de formule >C_{α}-S-CH=CH- (h), >C_{α}CH=CH-S- (i) ou >C_{α}-CH=CH-CH=CH- (j) éventuellement substitué par un halogène, un trifluorométhyle, un alcoyle inférieur, un alcoxy inférieur, un nitro, un amino ou un mono- ou di(alcoyle inférieur)amino et la ligne pointillée représente une liaison supplémentaire, et les radicaux décrits comme inférieurs possèdent au plus 7 atomes de carbone.

2. Composés selon la revendication 1, dans lesquels Q¹ représente avec l'atome d'azote le groupe de formule >N-CH₂CH₂- (a) ou >N-CH=CH- (c).

3. Composés selon la revendication 1 ou 2, dans lesquels Ra représente un groupe phényle éventuellement substitué par un m-halogène ou un m-trifluorométhyle.

4. Composés selon la revendication 3, dans lesquels Ra représente un phényle.

5. Composés selon l'une des revendications 1-4, dans lesquels Rb et Rc représentent avec l'atome de carbone désigné par α un groupe de formule >C_{α}-S-CH=CH- ou >C_{α}-CH=CH-CH=CH- éventuellement substitué par un halogène et la ligne pointillée représente une liaison supplémentaire.

6. Composés selon la revendication 5, dans lesquels Rb et Rc représentent ensemble avec l'atome de carbone désigné par α le groupe de formule >C_{α}-S-CH=CH- ou >C_{α}-CH=CCl-CH=CH-.

7. Hydroxyde de 5,6-dihydro-3-hydroxy-2-phénylthiazole[3,2-a]thiéno[2,3-c]pyridinium (sel interne).

8. Hydroxyde de 9-chloro-2-phényl-5,6-dihydro-3-hydroxythiazolo[2,3-a]isoquinolinium (sel interne).

9. Procédé de préparation de composés selon l'une des revendications 1-8, caractérisé en ce qu'on fait réagir un composé de formule générale 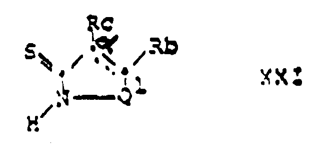 dans laquelle Q¹ , Rb, Rc et la ligne pointillée ont la signification donnée dans la revendication 1, avec un composé de formule générale
Ra-CHX²-COX¹ XXII ou 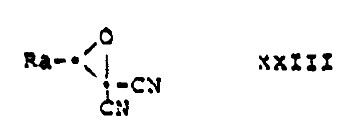
dans laquelle X¹ et X² représentent chacun un halogène et Ra a la signification donnée dans la revendication 1.

## Revendications (Revendications pour l'(les) Etat(s) contractant(s) suivant(s): AT)

1. Procédé de préparation de composés de formule générale 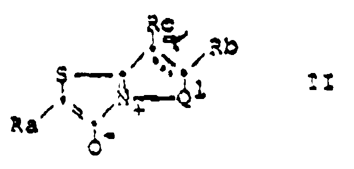 dans laquelle Q¹ représente avec l'atome d'azote un groupe de formule >N-CH₂CH₂- (a), >N-CH₂CH₂CH₂- (b), >N-CH=CH- (c), >N-CH₂-CH=CH- (d), >N-CH₂-S(O)ₚ- (e), >N-CH₂CH₂-S(O)ₚ- (f) ou >N-CH=CH-S(O)ₚ- (g), p représente le nombre 0, 1 ou 2, Ra un groupe phényle, pyridyle ou thiényle éventuellement substitué par un halogène, un trifluorométhyle, un nitro, un alcoyle inférieur ou un alcoxy inférieur, Rb et Re représentent ensemble avec l'atome de carbone désigné par α un groupe de formule >C_{α}-S-CH=CH- (h), >C_{α}CH=CH-S- (i) ou >C_{α}-CH=CH-CH=CH- (j) éventuellement substitué par un halogène, un trifluorométhyle, un alcoyle inférieur, un alcoxy inférieur, un nitro, un amino ou un mono- ou di(alcoyle inférieur)amino et la ligne pointillée représente une liaison supplémentaire, et les radicaux décrits comme inférieurs possèdent au plus 7 atomes de carbone,
caractérisé en ce qu'on fait réagir un composé de formule générale 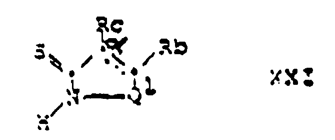 dans laquelle Q¹ , Rb, Rc et la ligne pointillée ont la signification donnée ci-dessus avec un composé de formule générale
Ra-CHX²-COX¹ XXII ou 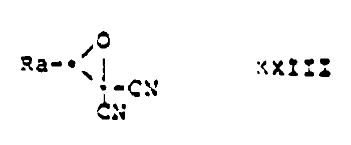
dans laquelle X¹ et X² représentent chacun un halogène et Ra a la signification donnée ci-dessus.

2. Procédé selon la revendication 1, dans lequel Q¹ représente avec l'atome d'azote le groupe de formule >N-CH₂CH₂- (a) ou >N-CH=CH- (c) .

3. Procédé selon la revendication 1 ou 2, dans lequel Ra représente un groupe phényle éventuellement substitué par un m-halogène ou un m-trifluorométhyle.

4. Procédé selon la revendication 3, dans lequel Ra représente un phényle.

5. Procédé selon l'une des revendications 1-4, dans lequel Rb et Rc forment avec l'atome de carbone désigné par α un groupe de formule >C_{α}-S-CH=CH- ou >C_{α}-CH=CH-CH=CH- éventuellement substitué par un halogène et la ligne pointillée représente une liaison supplémentaire.

6. Procédé selon la revendication 3, dans lequel Rb et Rc représentent ensemble avec l'atome de carbone désigné par α le groupe de formule >C_{α}-S-CH=CH- ou >C_{α}-CH=CCl-CH=CH-.

7. Procédé selon la revendication 1, caractérisé en ce qu'on prépare l'hydroxyde de 5,6-dihydro-3-hydroxy-2-phénylthiazolo[3,2-a]thiéno[2,3-c]pyridinium (sel interne).

8. Procédé selon la revendication 1, caractérisé en ce qu'on prépare l'hydroxyde de 9-chloro-2-phényl-5,6-dihydro-3-hydroxythiazolo[2,3-a]isoquinolinium (sel interne).
